# EUROPÄISCHE PATENTANMELDUNG

(11) **EP 4 064 287 A1**
(43) Veröffentlichungstag der Anmeldung: **28.09.2022**
(21) Anmeldenummer: 22020128.9
(22) Anmeldetag: 24.03.2022
(51) Int. Cl.: G16H 10/60, G16H 50/20, G16H 50/70

(54) **VERFAHREN FÜR DIE VORHERSAGE VON KRANKHEITEN VON NUTZTIEREN**

(30) Priorität: 24.03.2021 AT 632021
(71) Anmelder: ZuchtData EDV Dienstleistungen GmbH, 1200 Wien (AT); Rinderzucht Austria, 1200 Wien (AT); Medizinische Universität Wien, 1090 Wien (AT); Universität für Bodenkultur Wien, 1180 Wien (AT)
(72) Erfinder: LASSER, Jana, 8010 Graz (AT); MATZHOLD, Casper, 8062 Kumberg (AT); KLIMEK, Peter, 1130 Wien (AT); FÜRST-WALTL, Birgit, 8051 Graz (AT); EGGER-DANNER, Christa, 3343 Hollenstein (AT); STEININGER, Franz, 3921 Langschlag (AT)

(57) **Zusammenfassung**

Die Erfindung betrifft ein Verfahren für die Vorhersage einer Krankheit von Nutztieren wobei das Auffinden einer Prognoseregel durch Anwendung von maschinellem Lernen an die Nutztiere betreffenden Datensätzen in einer Datenbank erfolgt. Das maschinelle Lernen wird dabei in zwei zeitlich nacheinander stattfindenden und zueinander unterschiedlichen Arbeitsstadien angewendet, wobei in dem früher stattfindenden Arbeitsstadium an den umfangreicheren Datensätzen maschinell gelernt wird als in dem später stattfindendem Arbeitsstadium, wobei Datensätze an denen im späteren Arbeitsstadium maschinell gelernt wird durch Weglassung von Parametern verkleinert sind, wobei die weggelassenen Parameter solche sind, bei denen im früheren Arbeitsstadium erkannt wurde, dass ihr Beitrag zur Prognosegüte unter einer festgelegten Mindestgrenze liegt.

## Beschreibung

Zur Nutztierhaltung sind computerunterstützte Verfahren bekannt mit denen für individuelle Nutztiere eine Aussage über die Wahrscheinlichkeit für das zukünftige Eintreten von bestimmten Krankheiten berechnet wird. Beispielsweise kann damit ein Wahrscheinlichkeitswert generiert werden, welcher für ein einzelnes bestimmtes Nutztier aussagt wie hoch die Gefahr ist, dass innerhalb eines bestimmten Zeitraumes Ketose auftritt. Ein wesentliches Ziel derartiger Verfahren ist es, durch Berücksichtigung diverser Risikofaktoren Erkrankungen bzw. deren Entstehung frühzeitig zu erkennen, um therapierende oder vorbeugende Maßnahmen so schnell wie möglich starten zu können.

Durch die WO 0128415 A1 werden ein Verfahren und ein System vorgestellt, gemäß welchen eine Datenbank laufend Daten über Biographie und körperliche und gesundheitliche Merkmale von individuellen Tieren sammelt, und aus diesen Daten für Kunden auf Anforderung und Zahlung hin, einen Bericht generiert und ausliefert, welcher zu einem einzelnen bestimmten Tier Aussagen über die Wahrscheinlichkeiten von Störungen und Krankheiten und die zu erwartende Lebensdauer beinhaltet. Die Eingaben bezüglich körperlicher Merkmale betreffen vorwiegend genetische Daten und Daten über Konzentrationen von Substanzen in Körperflüssigkeiten oder Gewebeproben der Tiere. Die Datenbank erstellt dabei die Prognosen nicht einfach nur nach einem statischen unveränderbaren Schema, sondernd sie verbessert auch das Schema laufend unter Miteinbeziehung der Daten über jeweils neu hinzugekommene tatsächliche Ereignisse, sodass also ein lernendes System vorliegt. Kommunikation mit der Datenbank erfolgt typischerweise von individuellen Computern aus über Internet.

Die WO 2015/176027 A1 beschreibt ein computerimplementiertes Verfahren für das Vorschlagen eines Behandlungsplanes für eine Tierherde, wobei der Behandlungsplan vorwiegend die Fütterung aber auch die Verabreichung von Medikamenten betrifft. Das Verfahren verwendet eine Datenbank in welcher Daten dazu gespeichert sind, wie in der Vergangenheit Tierherden die bestimmten Umgebungsbedingungen ausgesetzt waren, und bestimmte Krankheiten hatten, mit bestimmten Medikationen behandelt wurden, und wie der Erfolg dazu war. Zur Anwendung an einer Herde wird aus einer an wenigen Tieren der Herde erstellten Diagnose ein Behandlungsplan für alle Tiere der Herde vorgeschlagen. Durch die Datenbank werden dazu jene Einträge zu anderen Herden, welche bezüglich Krankheiten, die Tiere beschreibenden Parametern und Umgebungsbedingungen am besten übereinstimmenden, gefunden, und die die dazu passenden, bekanntermaßen erfolgreichen Behandlungspläne, erneut vorgeschlagen.

Durch die US 2007/0112598 A1 wird vorgeschlagen, Gesundheitsdaten, die Menschen betreffen, und aus diversen unterschiedlichen Datenquellen stammen, in einer Datenbank zu sammeln und diese Daten, welche nicht bereinigt (harmonisiert) sein müssen, mittels maschinellem Lernen nach Regeln zu durchsuchen, aus denen sich Gesundheitsprognosen - und diesbezügliche Ratschläge - für einzelne Menschen ableiten lassen. Gemäß einem Geschäftsmodell dazu, können Menschen aus der Datenbank Prognosen zu ihrer eigenen gesundheitlichen Entwicklung abfragen - nachdem sie relevante Daten über sich selbst eingegeben haben. Auf nähere Umstände des maschinellen Lernens wird nicht eingegangen.

Die der Erfindung zu Grunde liegende Aufgabe besteht darin, ein verbessertes Verfahren für die Angabe der Wahrscheinlichkeit des zukünftigen Auftretens bestimmter Krankheiten an individuellen Nutztieren bereitzustellen. Die Verbesserungen sollen darin bestehen, dass besser als bisher die Breite von ohnedies anfallenden Daten genutzt und in weiterer Folge reduziert wird ohne dass der Verarbeitungsaufwand für die Daten ausufert, und dass ohne Verlust an Prognosegüte mit einem geringeren Umfang an Eingangsdaten - oder mit lückenhaften Eingangsdaten - das Auslangen gefunden werden kann.

Für das Lösen der Aufgabe wird von einem an sich bekannten Ansatz gemäß den folgenden Merkmalen ausgegangen:
- Es wird eine computerbasierte Datenbank angelegt, welche eine Vielzahl von Datensätzen beinhaltet, welche jeweils ein Nutztier betreffen, welches tatsächlich existiert hat oder noch existiert.
- Die Parameter der Datensätze betreffen eine breite Palette von Daten bezüglich gesundheitlicher Zustände des Nutztieres sowie Daten bezüglich eindeutiger oder mutmaßlicher Einflussfaktoren auf gesundheitliche Zustände des Nutztieres.
- Aus der Vielzahl von Datensätzen werden durch maschinelles Lernen (computerbasierte statistische Analysen) statistische Abhängigkeiten des Auftretens bestimmter Krankheiten (Ausgangsparameter) vom Vorliegen bestimmter Werte anderer Parameter (Eingangsparameter) untersucht und quantifiziert.
- Die erkannten Abhängigkeiten werden dazu verwendet, zu individuellen Nutztieren, zu denen Eingangsparameter in die Datenbank einzugeben sind, die Wahrscheinlichkeit für das zukünftige Auftreten bestimmter Krankheiten zu prognostizieren.

Als erfindungsgemäße Verbesserung dazu wird vorgeschlagen, das maschinelle Lernen in zwei unterschiedlichen Arbeitsstadien anzuwenden, wobei die Datensätze an welchen im zweiten Arbeitsstadium maschinell gelernt wird, gegenüber jenen an welchem im ersten Arbeitsstadium maschinell gelernt wird, durch Weglassung von Parametern verkleinert sind, wobei die weggelassenen Parameter solche sind, bei denen im ersten Arbeitsstadium erkannt wurde, dass ihr Beitrag zur Prognosegüte unter einer festzulegenden Mindestgrenze liegt.

Die Erfindung wird im Folgenden an Hand von Zeichnungen und einem Anwendungsbeispiel näher erläutert.
Fig. 1 zeigt ein Flussdiagramm, welches den Kern des erfindungsgemäßen Verfahrens für das Finden von Prognoseregeln für die Angabe der Wahrscheinlichkeit des zukünftigen Auftretens einer Krankheit an Nutztieren veranschaulicht.
Fig. 2 veranschaulicht in einem Flussdiagramm eine beispielhafte Anwendung des erfindungsgemäßen Verfahrens.

In den Zeichnungen bezeichnen gleiche Bezugsziffern den gleichen Gegenstand.

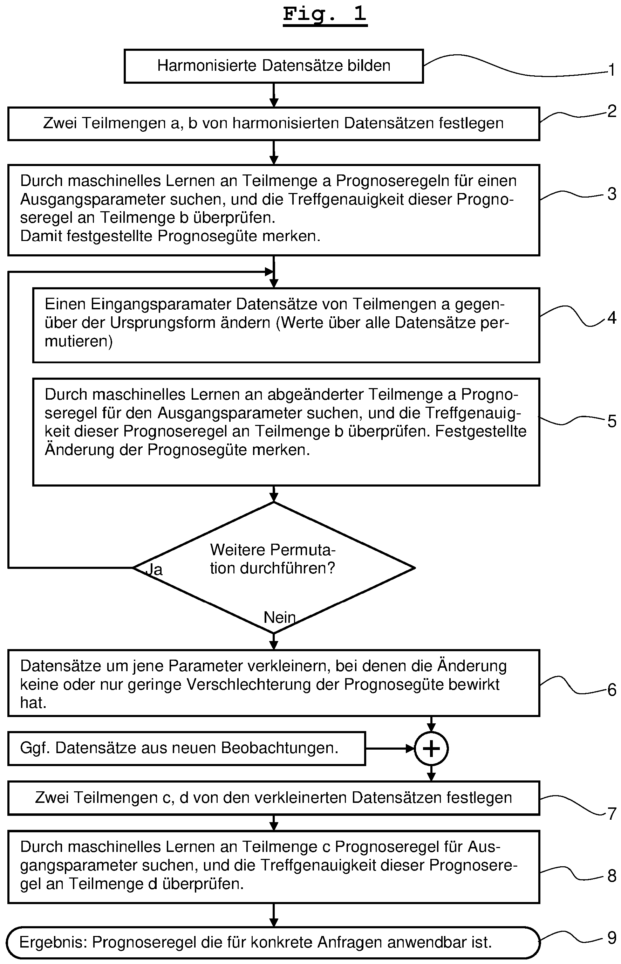

In Fig. 1 bedeuten die eckigen umrandeten Bereiche Arbeitsschritte, die im Allgemeinen durch einen Computer durchzuführen sind. Die Pfeile bedeuten im Allgemeinen Informationen die zwischen jenen Arbeitsinstanzen, welche die einzelnen Arbeitsschritte durchführen, weitergeben werden. Die Arbeitsinstanzen sind jeweils durch Software, welche in einem Computer läuft, realisiert. Wahlweise können verschiedene Arbeitsinstanzen in unterschiedlichen Computern untergebracht sein, oder auch alle Arbeitsinstanzen am selben Computer.

Schritt 1 gemäß Fig. 1 betrifft das Bilden von harmonisierten Datensätzen aus unterschiedlich strukturierten Rohdatensätzen, die unterschiedliche Einflussfaktoren auf die Gesundheit von Nutztieren abbilden. Das umfasst das Anlegen von harmonisierten Datensätzen, das Auslesen von Daten von Datensätzen von Rohdaten und das Einlesen der Daten in die harmonisierten Datensätze. All dies erfolgt typischerweise automatisiert durch einen Computer, an welchen die Rohdaten geliefert werden.

Die besagten Rohdaten fallen in und um jene landwirtschaftlichen Betriebe an, welche die Nutztiere halten. Diese Rohdaten enthalten sowohl Aussagen welche nur genau ein individuelles Nutztier betreffen, wie beispielsweise Identitätskennzeichnung des Nutztieres, Historie, ggf. aktuelle Merkmale des Nutztieres, als auch Daten über Einrichtung und Arbeitsweisen des Betriebes zu dem das Nutztier gehört als Ganzes, sowie auch Daten über die Umwelt des Betriebes wie Klima- und/oder Wetterdaten und die Information über die Seehöhe. Diese Rohdaten werden naturgemäß in der Regel unterschiedlich oft aktualisiert, und sind im Allgemeinen nicht in einer einzelnen Datenbank gesammelt, sondern in voneinander separaten sogenannten Urdatenbanken, die zu so unterschiedlichen Institutionen wie beispielsweise einem Rinderzuchtverband und einer nationalen Wetterbehörde gehören können.

Jeder harmonisierte Datensatz bezieht sich genau auf ein individuelles Nutztier, beispielsweise eine Milchkuh. Der harmonisierte Datensatz enthält Daten sowohl über unmittelbare Eigenschaften und die Historie des Nutztieres selbst, als auch Daten über den Betrieb zu welchem dieses Nutztier gehört und auch über die Umwelt des Betriebes. All diese Informationen liegen natürlich nicht in Prosa vor, sondern in einer strukturierten Form, welche als Tabelle darstellbar ist, wobei die gesamte Tabelle dem Nutztier zuordenbar ist, die Spaltenüberschriften die Bezeichnung einzelner Parameter sind, und die Zeilen einzelnen Beobachtungen (Messungen von Zuständen) entsprechen, die jeweils einem Nutztier zuordenbar sind. Je mehr Beobachtungen von einem Nutztier gemacht werden, desto mehr Zeilen hat die besagte Tabelle. Der Informationstyp der einzelnen Parameter ist binär (ja/nein), kategorial (begrenzte ganzzahlige Anzahl von Auswahlmöglichkeiten) oder numerisch.

Bei einem im Rahmen der Entwicklung des vorliegenden Verfahrens konkret durchgeführten Beispiel, bei welchem die Nutztiere Milchkühe sind, hat ein harmonisierter Datensatz 120 Parameter.

Für das Finden einer Prognoseregel für die Vorhersage der Wahrscheinlichkeit des Eintretens einer einzelnen bestimmten Krankheit von Nutztieren werden in Schritt 2 gemäß Fig. 1 aus der Gesamtmenge von harmonisierten Datensätzen zwei Teilmengen a, b von harmonisierten Datensätzen ausgewählt, welche bezüglich der Häufigkeit des Auftretens der Krankheit beide hinreichend repräsentativ für statistische Analysen sind. Dieses Auswählen ist deswegen erforderlich, weil die Gesamtmenge von harmonisierten Datensätzen im Allgemeinen nicht die wahren Häufigkeitsverhältnisse über das Auftreten der Krankheit widerspiegelt, und somit zufällige statistische Schwankungen von der maschinellen Lerneinheit fälschlich als funktioneller Zusammenhang erkannt werden könnten. Ein Parameter der harmonisierten Datensätze a, b ist die Information ob bei dem betreffenden Nutztier jene Krankheit, für welche Prognoseregeln zu finden sind, aufgetreten ist oder nicht.

In Schritt 3 gemäß Fig. 1 wird an der Teilmenge a von harmonisierten Datensätzen durch maschinelles Lernen ganz konkret eine Prognoseregel für die Vorhersage der Wahrscheinlichkeit des Eintretens der interessierenden Krankheit von Nutztieren gesucht, und die zur Teilmenge a gefundene Prognoseregel wird an der Teilmenge b verifiziert. Für dieses Verifizieren werden die Informationen über das Auftreten bzw. Nicht-Auftreten der Krankheit bei den harmonisierten Datensätzen der Teilmenge b entfernt und an den Verbleibenden Daten für jeden dieser Datensätze die zur Teilmenge a gefundene Prognoseregel angewandt, also je Datensatz der Teilmenge b eine Prognose bezüglich des Auftretens der Krankheit erstellt. Dann wird je Datensatz verglichen wie die erstellte Prognose mit der Wirklichkeit übereinstimmt, und aus der Summe dieser Vergleiche wird ein Zahlenwert generiert, welcher die statistische Verlässlichkeit der Prognoseregel, also die Prognosegüte quantifiziert. Beispielsweise kann der die Prognosegüte quantifizierende Zahlenwert der sog. F1-Score sein, das ist ein zwischen 0 und 1 liegendes Maß für die Verlässlichkeit einer statistischen Prognose, wobei der Wert 1 für die höchstmögliche Verlässlichkeit steht. Dabei errechnet sich der F1-Score als das harmonische Mittel von Sensitivität und Spezifität. Dabei quantifiziert die Fähigkeit der Prognose, die Tiere mit der Krankheit korrekt zu identifizieren, und die Spezifität quantifiziert die Fähigkeit der Prognose, die Tiere ohne die Krankheit korrekt zu identifizieren.

Für das besagte maschinelle Lernen sind in der Fachwelt mehrere computerbasierte Algorithmen bekannt, weshalb hier nicht auf deren Funktionsweise im Detail darauf eingegangen wird. Im Rahmen der Erfindung wurden die drei Algorithmen für maschinelles Lernen, nämlich
- logistische Regression,
- Random-Forests-Klassifikatoren und
- Gradient-Boost-Entscheidungsbäume (XGBoost)
angewandt und prinzipiell für geeignet befunden.

In Schritt 4 gemäß Fig. 1 wird an den harmonisierten Datensätzen der Teilmenge a ein Eingangsparameter (also nicht jener Parameter der das Vorhandensein der zu prognostizierenden Krankheit unmittelbar aussagt) verändert. Konkret wird die Zuordnung der Werte zu den einzelnen Datensätzen stehen, willkürlich vermengt, bei gleichbleibender Reihenfolge der Datensätze also die Reihenfolge der Werte des Parameters permutiert.

Gemäß Abwandlungen des Verfahrens können auch alle Parameter einer Gruppe von Parametern, die alle den gleichen Lebensbereich eines Nutztieres betreffen, gleichzeitig in dieser Weise verändert werden. Typischerweise umfasst solch eine Gruppe von Parametern alle Parameter, zu denen vor der Harmonisierung (Schritt 1) die Informationen von einer der Urdatenbanken gemeinsam bereitgestellt werden.

In Schritt 5 wird mit den gemäß Schritt 4 veränderten harmonisierten Datensätzen der Teilmenge a der Schritt 3 - Erstellen einer Prognoseregel für die Krankheit und Verifizieren dieser Regel an den Datensätzen der Teilmenge b - wiederholt. Im Allgemeinen wird beim Verifizieren der damit erreichbaren Prognosegüte eine andere Prognosegüte festgestellt werden, als sie in Schritt 3 festgestellt wurde.

Die Arbeitsschritte 4 und 5 werden mit jeweils anderem, gegenüber der Ursprungsform veränderten Parameter (bzw. Parametern) in den Datensätzen der Teilmenge a wiederholt. Ergebnis all dieser Wiederholungen ist eine Tabelle, welche aussagt, wie stark sich die Veränderung einzelner Eingangsparameter - bzw. einzelner Gruppen von gemeinsam veränderten Eingangsparametern - in den harmonisierten Datensätzen auf die Prognosegüte bezüglich der interessierenden Krankheit auswirkt.

In Arbeitsschritt 6 werden Datenmengen aus verkleinerten Datensätzen gebildet. Die verkleinerten Datensätze werden durch Weglassung von Parametern von den - in Arbeitsschritt 1 erstellten - harmonisierten Datensätzen gebildet, sind ansonsten aber gleich aufgebaut wie die harmonisierten Datensätze. Dabei sind die weggelassenen Parameter jene Eingangsparameter, zu denen bei den Durchläufen durch die Arbeitsschritte 4 und 5 festgestellt wurde, dass ihr Beitrag zur Prognosegüte bezüglich der interessierenden Krankheit unterhalb einer festgelegten Mindestgrenze liegt.

Mit der aus den verkleinerten Datensätzen bestehenden Datenmenge werden in Arbeitsschritt 7 analog zu Arbeitsschritt 2 wiederum Teilmengen c, d von verkleinerten Datensätzen gebildet. Analog zu Arbeitsschritt 3 wird in Arbeitsschritt 8 nun mit verkleinerten Datensätzen an Hand der Teilmengen c, d eine Prognoseregel für die interessierende Krankheit durch maschinelles Lernen erarbeitet.

Die Folge der Arbeitsschritte 7 und 8 ist deutlich weniger aufwändig als die vergleichbare Folge der Arbeitsschritte 2 und 3, da sie mit verkleinerten Datensätzen erfolgt. Wenn neue Daten anfallen, können diese Daten - wie in Fig. 1 zwischen den Arbeitsschritten 6 und 7 angedeutet - in jene Datenmenge eingepflegt werden, welche in den Arbeitsschritten 7 und 8 verarbeitet wird. Die neu hinzukommenden Daten brauchen dabei nur Angaben zu den Parametern der gegenüber den harmonisierten Datensätzen verkleinerten Datensätze enthalten.

Wie aus dem unten beschriebenen Anwendungsbeispiel hervor geht, können oftmals sämtliche Parameter eines bestimmten Rohdatensatzes weggelassen werden ohne signifikante Reduktionen in der Prognosegüte in Kauf nehmen zu müssen. Jedoch sind es für unterschiedliche Krankheiten typischerweise unterschiedliche Rohdatensätze, die weggelassen werden können. Mit dem hier beschriebenen Verfahren kann nun eruiert werden, für welche Krankheit welche Rohdatensätze weggelassen werden können und in weiterer Folge nicht zum Zwecke der Krankheitsvorhersage erfasst und dokumentiert werden brauchen, wodurch sich der Arbeitsaufwand erheblich reduzieren kann.

Das Ergebnis des Arbeitsschrittes 8 ist eine Prognoseregel 9, welche besagt, wie die Wahrscheinlichkeit des Auftretens der interessierenden Krankheit von den sonstigen Parametern (Eingangsparametern) in den verkleinerten Datensätzen abhängig ist. Dabei sind die Eingangsparameter jene, welche in den Arbeitsschritten 2 bis 5 als maßgeblich für die jeweilige Prognosegüte erkannt wurden.

Die Prognoseregel 9 entspricht in angeschriebener Form im Wesentlichen einer nicht all zu großen Tabelle oder Matrix. Bestimmungsgemäß wird die Prognoseregel dazu verwendet, für ein konkretes Nutztier, die Information generieren, wie hoch die Wahrscheinlichkeit des Auftretens der interessierenden Krankheit in der Zukunft ist. Dieses Bestimmen erfolgt wiederum in einer Arbeitsinstanz eines Computers, in welcher die Prognoseregel und ein Programm, welches besagt wie mit der Prognoseregel umzugehen ist gespeichert ist, und an welche für konkrete Prognosen die das konkrete Nutztier betreffenden Eingangsparameter (des verkleinerten Datensatzes) einzugeben sind.

Verglichen mit dem zu ihrer Entstehung verarbeiteten Datenvolumen ist das Datenvolumen der Prognoseregel 9 deutlich geringer. Auch das für ihre Anwendung erforderliche Computerprogramm ist relativ einfach und klein. Deswegen kann das letztendliche Erstellen von konkreten Prognosen für konkrete Nutztiere auch durch einen ganz einfachen Computer, beispielsweise ein Smartphone, realisiert werden.

Die Arbeitsschritte 2 bis 8 sind für jede interessierende Krankheit zu durchlaufen, bis letztendlich für jede interessierende Krankheit eine eigene Prognoseregel 9 vorliegt.

Bei der praktischen Anwendung des Verfahrens werden auch die Arbeitsschritte 9 bis 6, welche in Summe sehr aufwändig sind, wiederholt durchlaufen, allerdings in relativ seltenen Zyklen, beispielsweise alle paar Monate. Ein Durchlauf sollte erfolgen, wenn eine erhebliche Menge neuer Daten vorliegt. Besonders zu empfehlen ist ein neuer Durchlauf, wenn eine weitere Quelle von Rohdaten erschlossen wurde. Zwingend ist ein neuer Durchlauf erforderlich, wenn die Form der harmonisierten Datensätze geändert wird, beispielsweise indem weitere Parameter hinzugenommen werden.

Indem im Zuge des erfindungsgemäßen Verfahrens der Beitrag jedes einzelnen Parameters zur Prognosegüte berechnet wird, und im weiteren Verlauf nur jene Parameter für das Erstellen von Prognoseregeln verwendet werden, welche zur jeweiligen Prognosegüte einen spürbaren Beitrag leisten, kann zu Beginn des Verfahrens von einer wesentlich größeren Parameterzahl und einer wesentlich breiteren Vielfalt an Parametern ausgegangen werden, als dies bisher als praktikabel erschien. Damit wird gegenüber bisherigen Methoden nicht nur eine deutlich breitere und umfassendere Information über die einzelnen Nutztiere und deren Lebensbedingungen abgebildet, es werden teilweise auch bessere Prognosen erstellt und es kann besser nachvollziehbar und praktisch ohne Verlust von Prognosegüte für aktuelle Prognosen auf die Erhebung und Verarbeitung ausschließlich jener Parameter eingeschränkt werden, welche sich nennenswert auf die Prognosegüte auswirken.

Die Anwendung derart breiter Datensätze und die vertiefte Verarbeitung führen neben der erwarteten Bestätigung von auch bisher schon bekannten statistischen Zusammenhängen zu den folgenden überraschenden und vorteilhaft anwendbaren Erkenntnissen:
a) Trotz der hohen Parameteranzahl kann auch dann, wenn tausende von Datensätze vorliegen, mit durchaus aufbringbaren Ressourcen bezüglich Hard- und Software und Zeit eine automatische statistische Analyse der Daten vorgenommen werden, welche Korrelationen zwischen verschiedensten Eingangsparametern und auch Kombinationen von mehreren Eingangsparametern einerseits und einzelnen Ausgangsparametern andererseits, überprüft und quantifiziert, sodass ggf. vorhandene starke Korrelationen zuverlässig erkannt werden.
b) Am Beispiel Milchkühe wird für eine Reihe von Krankheiten gezeigt, wie die Wahrscheinlichkeit ihres Auftretens als Folge einer speziellen Kombination von mehreren Vorbedingungen aus höchst unterschiedlichen Merkmalskategorien erstmals erkennbar gemacht werden kann.
c) Es lässt sich für jede der Erkrankungen angeben, wieviel Prozent der Prognosegüte jeweils von den unterschiedlichen Kategorien von Parametern stammt.

Durch Weglassung von als unbedeutend für die jeweilige Prognosegüte erkannten Parametern können Prognoseverfahren im Sinne einer Datenminimierung optimiert werden, d.h. dass Ressourcen zur Datenerfassung auf diejenigen Merkmale konzentriert werden können, die am maßgeblichsten für die interessierende Vorhersage sind.

Jene Parameter der harmonisierten Datensätze, welche einen nennenswerten Beitrag zur Güte der Prognose einer Erkrankungswahrscheinlichkeit liefern, sind gleichzeitig auch genau jene Parameter, deren Wert sich spürbar auf die Erkrankungswahrscheinlichkeit auswirkt, sodass durch Veränderung ihres Wertes auch die Erkrankungswahrscheinlichkeit verändert werden kann. Indem diese Parameter für die interessierenden Krankheiten durch die Schritten 2 bis 6 nachvollziehbar Identifiziert werden, ist auch die Information gewonnen, an welchen Parametern ggf. Veränderungen vorzunehmen sind, um eine Erkrankungswahrscheinlichkeit zu verringern. Die Richtung in welcher ggf. ein Parameter verändert werden muss, um ein Risiko zu vermindern, kann beispielsweise durch Eingabe eines veränderten Parameterwertes in die Prognoseberechnung festgestellt werden. Durch Analyse der in Schritt 7 definierten Datenmengen c, d durch ein reduziertes logistisches Regressionsmodell (das ist ein spezieller maschineller Lernvorgang) können allgemeine Regeln gefunden werden, welche die Aussage generieren, welche Richtung der Veränderung eines Eingangsparameters welche Richtung der Veränderung des Ausgangsparameters bewirkt.

Das erfindungsgemäße Verfahren ist im Bereich der Nutztierhaltung extrem vorteilhaft anwendbar, weil in diesem Bereich sehr viele und sehr heterogene Daten gesammelt wurden und werden, und weil ein großer Anteil der durch die Daten beschriebenen Umstände zwar einen statistischen, nicht aber einen einfach erkennbaren eindeutigen kausalen Zusammenhang mit Erkrankungsrisiken hat. Das erfindungsgemäße Verfahren eignet sich bestens dazu aus dieser Gemengelage unter wirtschaftlich gut vertretbarem Aufwand, die für die Prognosen relevanten Informationen zu identifizieren und anzuwenden.

Die besagten, im Nutztierbereich anfallenden Daten lassen sich grob in zwei Kategorien einteilen, nämlich in tierbezogene Daten und in umweltbezogene Daten. Dabei sind die tierbezogenen Daten solche, welche unmittelbar nur ein einziges individuelles Nutztier beschreiben und deren Feststellung das Vorhandensein eines Nutztieres zwangsweise erfordert. Die umweltbezogenen Daten sind dabei solche, welche Bedingungen der Umgebung in welcher Nutztiere leben, beschreiben. Zu ersteren gehören beispielsweise Informationen über körperliche Merkmale, Alter, gesundheitliche Historie, Rasse, Milchleistung (bei Milchkühen) eines individuellen Nutztieres; sie ergeben ohne Nutztier keinen Sinn. Zu letzteren Daten gehören beispielsweise Arbeitsweisen und Einrichtungen des Betriebes in welchen Nutztiere gehalten werden, sowie Umgebungsbedingungen des Betriebes wie typischerweise Klimadaten. Diese letzteren Daten können - wenn sie individuellen Nutztieren zugeordnet werden wie das im harmonisierten Datensatz der Fall ist - für mehrere Nutztiere gleich sein, wenn diese Nutztiere gleichzeitig im gleichen Betrieb untergebracht sind.

Das erfindungsgemäße Verfahren ist dann besonders sinnvoll anzuwenden, wenn für die Prognose der Wahrscheinlichkeit des zukünftigen Auftretens einer interessierenden Krankheit an einem Nutztier sowohl tierbezogene Daten als auch umweltbezogene Daten als potentielle Einflussfaktoren auf interessierende Krankheiten in Betracht genommen werden. Es ist umso sinnvoller, je breiter die Vielfalt an in Betracht gezogenen Einflussfaktoren ist und/oder je größer die Anzahl der in Betracht Einflussfaktoren ist.

Ganz besonders sinnvoll ist das erfindungsgemäße Verfahren anwendbar, wenn die Nutztiere an denen es angewandt wird, Milchkühe sind. Das ist deswegen so, weil zu Milchkühen - vor allem im Rahmen der Überwachung von Milchleistung und Milchgüte - standardmäßig extrem viele und extrem vielfältige Daten erhoben werden, welche in Betracht zu ziehende Einflussfaktoren auf Erkrankungswahrscheinlichkeiten betreffen.

In einem beispielhaften Anwendungsfall für einen landwirtschaftlichen Betrieb, wird für eine Milchkuh, welche zu dem Betrieb gehört, eine Prognose benötigt, welche besagt, wie hoch die Gefahr diese Milchkuh ist, innerhalb eines nahen Zeitraumes an einer bestimmten Krankheit zu erkranken.

Dabei entspricht die besagte Krankheit einem Ausgangsparameter der Zuordnungsvorschrift 7. Der Betrieb teilt dazu auf elektronischem Weg mit Hilfe eines Abfrageformulars Anfragedaten 9 bezüglich der betreffenden Milchkuh an die Prognoseeinheit 8 mit. Die Anfragedaten beinhalten zumindest die Werte der gemäß Zuordnungsvorschrift 7 zum abgefragten Ausgangsparameter gehörenden Einflussparameter. Die Prognoseeinheit sucht zu den besagten Werten die gemäß Zuordnungsvorschrift 7 gehörenden Werte der zugehörigen Ausgangsparameter und sendet diese an den Betrieb 1 zurück - womit dieser die gewünschte Prognoseinformation erhalten hat.

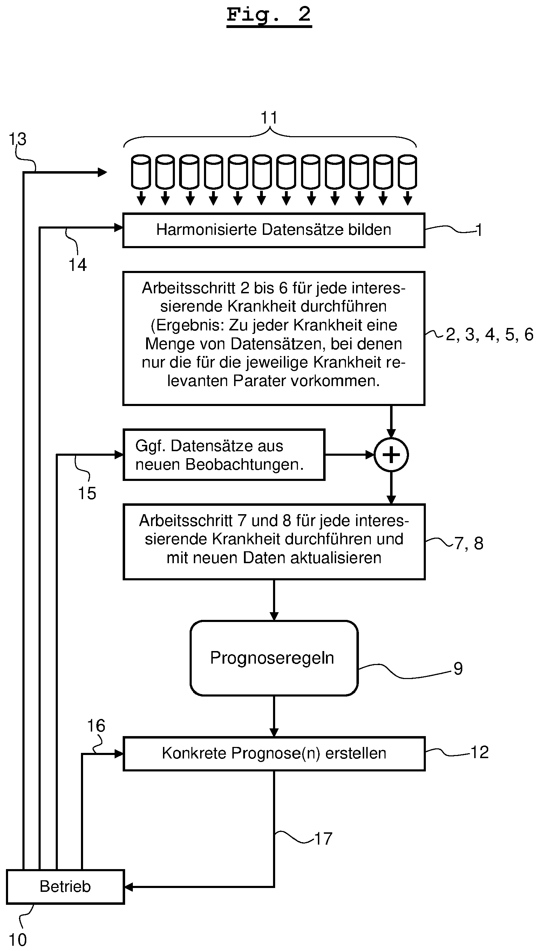

Am Beispiel gemäß Fig. 2 ist der Betrieb 10 ein Landwirtschaftsbetrieb welcher Nutztiere - beispielsweise Milchkühe - hält und bewirtschaftet. Bezüglich des Betriebes 10 werden mehr oder minder laufend Daten erhoben, welche in unterschiedlichen Urdatenbanken 11 gesammelt werden. Wie schon weiter oben erklärt können diese Urdatenbanken 11 zu ganz unterschiedlichen Organisationen gehören und an unterschiedlichen Orten positioniert sein. In Summe erfassen die Urdatenbanken eine breite Palette von Daten, welche zum Teil nur einem individuellen Nutztier zuordenbar sind, zum Teil aber besser dem gesamten Betrieb 10 zuordenbar sind, zum Teil die Umgebung des Betriebes betreffen. Aus den Daten der Urdatenbanken 11 werden in Arbeitsschritt 1 (zu Fig. 1 schon erläutert) harmonisierte Datensätze gebildet. Durch die zu Fig. 1 schon erläuterten Arbeitsschritte 2, 3, 4, 5 und 6 wird für jede interessierende mögliche Krankheit eines Nutztieres zu den einzelnen Parametern der harmonisierten Datensätze ein Wert ermittelt, welcher aussagt, wie sehr der betreffende Parameter die Prognosegüte bei der Angabe der Wahrscheinlichkeit für das zukünftige Auftreten dieser Krankheit an einem Nutztier beeinflusst. Unter Anwendung dieses Wissens werden in den Arbeitsschritten 7, 8 an Datenmengen, welche sich aus Datensätzen mit sinnvoll verringerter Parameterzahl zusammensetzen, Prognoseregeln 9 für die einzelnen möglichen Krankheiten erstellt. Diese Prognoseregeln 9 werden in Arbeitsschritt 12 dazu verwendet, zu einzelnen, konkreten Nutztieren, welche zum Betrieb 10 gehören, Prognosen zu erstellen, welche als Kernaussage die Information beinhalten, wie hoch die Wahrscheinlichkeit für das zukünftige Auftreten der interessierenden Krankheiten an dem Nutztier ist.

Das erfindungsgemäße Verfahren ist also keine Diagnosemethode für das Erkennen von bestehenden Krankheiten, sondern eine Methode für das Erkennen der Gefahr von ggf. drohenden Krankheiten.

Der Betrieb 10 kommuniziert - teilweise optional - auf drei Informationskanälen mit dem System für das erfindungsgemäße Verfahren.

Gemäß Kanal 13 liefert der Betrieb 10 wie schon erklärt diverse "Urdaten" an die Urdatenbanken 11.

Optional kann der Betrieb 10 auf Kanal 14 Daten schon ohne Umweg über die Urdatenbanken 11 an den Arbeitsschritt 1 liefern, in welchem harmonisierte Datensätze gebildet werden.

Optional kann der Betrieb 10 auf Kanal 15 Daten für die Erstellung von Prognoseregeln in den Arbeitsschritten 7, 8 liefern, wobei diese vom Betrieb 10 gelieferten Datensätze je interessierende Krankheit, nur die gemäß der Arbeitsschrittfolge 1, 3, 4, 5 und 6 gefundene reduzierte Anzahl von Parametern enthalten müssen.

Wenn der Betrieb 10 aktuelle Prognosen zur Erkrankungswahrscheinlichkeit von ganz konkreten Nutzieren haben will, so sendet er die für diese Prognose relevanten Daten auf Kanal 16 an die Arbeitsinstanz, welche den Arbeitsschritt 12 "Prognose erstellen" durchführt. Von dieser Arbeitsinstanz wird das Ergebnis, also die Prognose, auf Kanal 17 an den Betrieb 10 zurückgesandt.

Im Rahmen der Entwicklung des erfindungsgemäßen Verfahrens wurde und wird ein konkretes Projekt durchgeführt, in welchem das Verfahren angewendet wird. Dabei sind die Nutztiere zu denen Erkrankungswahrscheinlichkeiten zu prognostizieren sind, Milchkühe. Über das konkrete Projekt wird im Folgenden ergänzend auszugsweise berichtet:
Es wurden Daten von 5828 Milchkühen aus 166 Betrieben und 22923 Beobachtungen verwendet, die in den Jahren 2014 bis 2016 tatsächlich erhoben wurden. Der gesamte Umfang der Beobachtungen wurde in insgesamt 135 Parametern in den Urdatenbanken verzeichnet. Die 135 Parameter sind dabei insgesamt 11 sinnvoll von einander unterscheidbaren Lebensbereichen zugeordnet.

In den harmonisierten Datensätzen bedeutet jede Zeile eine Beobachtung. Eine sehr wesentliche Besonderheit ist der Umfang der jeweiligen Beobachtungen. Jede Beobachtung betrifft 135 Parameter (Merkmale), die insgesamt elf verschiedene Lebensbereiche des jeweiligen Nutztieres betreffen.

Die elf Lebensbereiche und die Anzahl der diese beschreibenden Parameter sind:

| **Lebensbereich (Parametergruppe)** | **Anzahl Parameter** |
|---|---|
| Alter | 2 |
| Diagnosen und Diagnoseherkunft | 5 |
| Fütterung | 40 |
| Laktationsstadium | 3 |
| Management | 20 |
| Milch-Indikatoren | 8 |
| physische Indikatoren | 5 |
| Rasse | 13 |
| Umwelt | 10 |
| Unterbringung | 24 |
| Zuchtwerte | 5 |

(Im Zuge des Projektes wurden zeitweise Parameter von einzelnen Lebensbereichen weggelassen oder zu einzelnen Lebensbereichen hinzugefügt.)

Beispielsweise umfasst(e) der Lebensbereich "Unterbringung" die folgenden Parameter:

| **Parameter** | **Datentyp** | **Ausprägungsmöglichkeiten:** |
|---|---|---|
| Liegeboxensystem | kategorial | 5 |
| Entmistung | kategorial | 4 |
| Fußboden im Freilaufbereich für Kälber | kategorial | 3 |
| Boden im Laufgang des Freilaufstalls für Kälber | kategorial | 4 |
| Einstreu im Freilaufstall für Kälber | kategorial | 3 |
| Entmistung im Freilaufstall für Kälber | kategorial | 3 |
| Freilaufstallsystem für Kälber | kategorial | 5 |
| Boden im Auslauf für laktierende Kühe | kategorial | 3 |
| Laufgangboden im Freilaufstall für laktierende Kühe | kategorial | 6 |
| Einstreu im Freilaufstall für laktierende Kühe | kategorial | 3 |
| Entmistung im Freilaufstall für laktierende Kühe | kategorial | 3 |
| Freilaufstallsystem für laktierende Kühe | kategorial | 3 |
| Boden im Freilandbereich für trockene Kühe | kategorial | 3 |
| Laufgangboden im Freilaufstall für trockene Kühe | kategorial | 5 |
| Einstreu im Freistall für trockene Kühe | kategorial | 3 |
| Entmistung im Freilaufstall für trockene Kühe | kategorial | 3 |
| Freilaufstallsystem für trockene Kühe | kategorial | 4 |
| Typ der Melkboxen | kategorial | 5 |
| Siloform | kategorial | 3 |
| Stallgestaltung | kategorial | 5 |
| Liegematten im Freistall für | kategorial | 2 |
| Kälber | | |
| Liegematten im Freilauf für laktierende Kühe | kategorial | 2 |
| Liegematten im Freistall für trockene Kühe | kategorial | 2 |

Beispielsweise umfasst(e) der Lebensbereich "Umwelt" die folgenden Parameter:

| **Parameter** | **Datentyp / Möglichkeiten** |
|---|---|
| Jahreszeit | kategorial / 4 |
| Seehöhe | numerisch |
| mittlere jährliche relative Feuchte | numerisch |
| Standardabweichung der jährlichen relativen Luftfeuchtigkeit | numerisch |
| mittlerer jährlicher Niederschlag | numerisch |
| Standardabweichung des Jahresniederschlags | numerisch |
| mittlere Jahrestemperatur | numerisch |
| Standardabweichung der Jahrestemperatur | numerisch |
| Anzahl der Starkwindtage | numerisch |
| Anzahl der Tage mit niedriger Temperatur | numerisch |

Beispielsweise umfasst(e) der Lebensbereich "Management (Betreuung)" die folgenden Parameter:

| **Parameter** | **Typ** | **Werte** |
|---|---|---|
| Häufigkeit der Klauenpflege | kategorial | zweimal pro Jahr / einmal pro Jahr / dreimal pro Jahr / nur bei lahmen Nutztieren |
| Melkabnahme | kategorial | keine / vorhanden einschließlich Nachmelktechnik / vorhanden |
| Trockene Kühe Gruppenhaltung | kategorial | getrennt / bei laktierenden Kühen / bei Kälbern |
| Kälber auf der Alm | kategorial | Ja / Nein |
| Laktierende Kühe auf der Alm | kategorial | Ja / Nein |
| Trockene Kühe auf der Alm | kategorial | Ja / Nein |
| Betriebsform Bio | kategorial | Ja / Nein |
| Kälber werden in einem Anbindestall gehalten | kategorial | Ja / Nein |
| Klauenpflege durch | kategorial | Ja / Nein |
| Landwirt | | |
| laktierende Kühe werden im Anbindestall gehalten | kategorial | Ja / Nein |
| Automatische Melkabschaltung | kategorial | Ja / Nein |
| Melkstimulation | kategorial | Ja / Nein |
| trockene Kühe in Anbindestallhaltung | kategorial | Ja / Nein |
| Weidegang von Kälbern | kategorial | Ja / Nein |
| Weidegang für laktierende Kühe | kategorial | Ja / Nein |
| Weidegang von trockenen Kühen | kategorial | Ja / Nein |
| Weidedauer von Kälbern | Numerisch | 20,7 ± 6,3 Tage |
| Weidedauer von laktierenden Kühen | Numerisch | 7,5 ± 5,23 Tage |
| Weidedauer der trockenen Kühe | Numerisch | 15,0 ± 9,1 Tage |
| Anzahl der Melkboxen | Numerisch | 7,13 ± 7,34 Stände |
| Melkvakuum | Numerisch | 42,1 ± 5,0 kPa |
| Jahresdurchschnitt der Herdenmilchleistung | Numerisch | 8728,0 ± 1528,0 kg |
| Herdengröße | Numerisch | 40,2 ± 18,8 Kühe |

Die Parameter umfassen also nicht nur unmittelbare tierspezifische Gesundheitsinformationen bzw. Informationen zu denen selbstverständlich bekannt ist, dass - und in welcher Weise - sie relevant für die Gesundheit von Nutztieren sind, sondern auch Informationen zu denen nicht ohne weiteres erkennbar oder erwartbar ist, dass und ggf. wie sie sich auf die Gesundheit von Nutztieren auswirken. Zu nennen sind beispielsweise Haltungs- und Managementbedingungen der Rinder, wie z.B. Stallarchitektur und Entmistungspraktiken; das Betriebsmanagement, wie z.B. Art und Dauer des Weidegangs; Informationen über das Melksystem; detaillierte Informationen über die Futterzusammensetzung - wie z.B. das Verhältnis von Rohfaser und Kraftfutter; Stoffwechselindikatoren; Informationen von Wetterdiensten - um Umweltfaktoren wie die durchschnittliche Niederschlagsmenge und Temperatur; Informationen über den aktuellen Laktationsstatus der Nutztiere sowie deren Parität.

Im Rahmen des Projektes sollen jene Parameter erkannt werden, die jeweilige Wahrscheinlichkeit für das Auftreten wirtschaftlich relevanter Milchviehkrankheiten - wie Ketose, Lahmheit und Mastitis - nennenswerten statistischen Einfluss haben, und diesen Einfluss auch zu quantifizieren.

Die folgende Tabelle zeigt für acht ausgewählte Krankheiten die Prognosegüte der im Projekt erreichten Vorhersagen für zwei unterschiedliche Datensätze. (Es wurde ein sog. XGBoost Algorithmus in der maschinellen Lerneinheit verendet.) Dabei umfasst der eine "vollständige Datensatz" Parameter zu elf Lebensbereichen, und der "reduzierte Datensatz" umfasst nur eine Teilmenge der Parameter des vollständigen Datensatzes, nämlich zu solchen Lebensbereichen, welche routinemäßig erfasst werden (Laktation, Alter, und Milchindikatoren), wobei jeweils einmal Daten aus der Trockenperiode mitberücksichtigt wurden, und einmal nicht.

### F1 Wert (mittels XGBoost)

| **Diagnose** | **Vollständiger Datensatz** | | **Reduzierter Datensatz** | |
|---|---|---|---|---|
| **Trockenperiode** | **mit** | **ohne** | **mit** | **ohne** |
| Anöstrus (= Stillbrunst) | 0, 67 | 0, 67 | 0, 67 | 0, 66 |
| Lahmheit | 0, 66 | 0, 68 | 0,63 | 0,65 |
| Ketose | 0, 61 | 0,57 | 0,54 | 0,51 |
| akute Mastitis | 0,43 | 0,49 | 0,49 | 0,36 |
| chronische Mastitis | 0,48 | 0,41 | 0,46 | 0,42 |
| Eierstockzysten | 0,59 | 0, 61 | 0,53 | 0,55 |
| periparturale Hypokalzämie | 0,55 | 0,39 | 0,50 | 0,39 |
| Metritis | 0,45 | 0,43 | 0,50 | 0,55 |

Es ist erkennbar, dass für einzelne Diagnosen (Anöstrus, akute Mastitis, chronische Mastitis, Hypokalzämie und Metritis) die Prognosegüte nicht merkbar abfällt oder sogar zunimmt, wenn statt des vollständigen Datensatzes nur der reduzierte Datensatz verwendet wird. Das heißt, dass in Anwendungen zur Prognose von Tiererkrankungen im Falle der oben genannten Erkrankungen die Erhebung von Informationen aus zusätzlichen Lebensbereichen nicht notwendig ist.

Um den Nutzen des Verfahrens weiter zu verdeutlichen, zeigt die nachfolgende Tabelle die Ergebnisse für den F1-Wert wenn die Daten zwar wie oben beschrieben harmonisiert werden, anstelle eines komplexeren maschinellen Lernverfahrens das Erkrankungsrisiko aber auf Basis der einfacheren logistischen Regression berechnet wird:

### F1 Wert mittels logistischer Regression

| **Diagnose** | **Vollständiger Datensatz** | | **Reduzierter Datensatz** | |
|---|---|---|---|---|
| **Trockenperiode** | **mit** | **ohne** | **mit** | **ohne** |
| Anöstrus *(= **Stillbrunst)*** | 0,74 | 0,73 | 0,39 | 0,40 |
| Lahmheit | 0,73 | 0,74 | 0,50 | 0,51 |
| Ketose | 0,52 | 0,44 | 0,16 | 0,05 |
| akute Mastitis | 0,49 | 0,48 | 0,20 | 0,17 |
| chronische Mastitis | 0,51 | 0,50 | 0,06 | 0,05 |
| Eierstockzysten | 0, 60 | 0,62 | 0,28 | 0,30 |
| periparturale Hypokalzämie | 0,48 | 0,38 | 0,27 | 0,00 |
| Metritis | 0,55 | 0,55 | 0,17 | 0,21 |

Im reduzierten Datensatz ist bei logistischer Regression eine deutlich herabgesetzte Prognosegüte im Vergleich zu XGBoost als maschinellem Lernverfahren erkennbar. Am vollständigen Datensatz sind die Unterschiede hingegen weniger deutlich. Vereinfacht gesagt, sind im vollständigen Datensatz so viele Informationen vorhanden, dass auch einfache statistische Modelle (Regression) eine entsprechende Prognosegüte liefern. Mittels fortgeschrittener maschineller Lernverfahren (z.B. XGBoost) kann jedoch auch mit reduzierten und daher weniger aufwändigen Datensätzen eine entsprechende Prognosegüte erreicht werden. Das vorliegende Verfahren erlaubt es also automatisiert herauszufinden, wie ein Datensatz reduziert werden kann wobei nur minimale Verluste in der Prognosegüte in Kauf genommen werden müssen.

Die nachfolgende Tabelle zeigt quantitative Auswirkungen, welche die jeweiligen, weiter oben erwähnten elf Lebensbereiche, auf die Prognosegüte der Vorhersage von fünf beispielhaften ausgewählten Krankheiten zeigen (Kumulative Beiträge der Permutationsmerkmalswichtigkeit für die elf Merkmalskategorien und fünf Krankheiten mit einem Vorhersage-Fl-Score von größer gleich 0,5.):

| **Lebensbereich** | **Lahmheit [%]** | **Anöstrus [%]** | **Ketose [%]** | **periparturale Hypokalzämie [%]** | **Metritis [%]** |
|---|---|---|---|---|---|
| **Alter** | 10,9 | 8,7 | 1 | 19,8 | 2,4 |
| **Diagnosen und Diagnoseherkunft** | 9,9 | 8,7 | 4,6 | 0,3 | 28,5 |
| **Fütterung** | 18,7 | 26,8 | 26,3 | 13,9 | 23,1 |
| **Laktationsstadium** | 0,4 | 1,5 | 6,3 | 25,9 | 2,1 |
| **Management** | 12,7 | 13,1 | 8, 6 | 4,2 | 4,7 |
| **Milch-Indikatoren** | 1,5 | 4,7 | 6,6 | 13,7 | 8,1 |
| **physische Indikatoren** | 4,0 | 2,7 | 5,8 | 5,0 | 5,2 |
| **Rasse** | 0,6 | 0,2 | 1,9 | 0,4 | 0,9 |
| **Umwelt** | 11,1 | 6,7 | 12,2 | 2,5 | 11,4 |
| **Unterbringung** | 28,2 | 30,8 | 23,4 | 12,5 | 10,6 |
| **Zuchtwerte** | 1,9 | 4,1 | 3,3 | 1,9 | 3,3 |

Beispielsweise kann für die Vorhersage von Anöstrus bei Milchkühen eine Prognosegüte (F1 Wert) von 78% erzielt werden bei einer Sensitivität von 64% und einer Spezifität von 99%. Diese Vorhersagen stammten zu 31% von Merkmalen der Kategorie "Behausung", zu 27% der Kategorie "Futtermittel" und zu 13% aus der Kategorie "Betriebsmanagement". Alle anderen Kategorien trugen weniger als 10% bei und waren damit für die Prognosegüte wenig maßgeblich.

Eine weitergehende Analyse der Risikofaktoren in diesen Kategorien ergab, dass die folgende Kombination von Merkmalen zu dem größten Risiko für Stillbrunst führte:
- Das Fehlen eines Zugangs zu Freiluftflächen im Stall erhöhte die Chancen auf Stillbrunst ("Odds Ratio", [OR]) um ein dreifaches (OR 2.95 mit 95%igem Konfidenzintervall [KI] von 2.50-3.49),
- eine Fütterung von Mais oder Maissilage mit einem ebenfalls dreifach erhöhten Risiko (OR 2.60 CI 2.05-3.29 bzw. OR 3.31 CI 2.80-3.92),
- sowie das Vorliegen eines Betriebs mit hoher jährlicher Milchleistung (OR 1.65, CI 1.50-1.80).

Parameterkombinationen dieser Art lassen sich für jede der betrachteten Krankheiten identifizieren und quantifizieren. Viele dieser Zusammenhänge erscheinen als in der Fachliteratur bisher noch nie beschrieben.

Weitere beispielhafte wertvolle Aussagen, die sich aus den Ergebnissen ablesen lassen, sind:
Im Allgemeinen dominiert keine einzelne Merkmalskategorie für eine Krankheit und zwei oder mehr Kategorien sind in der Regel notwendig, um mehr als 50 % der Beiträge zur Permutationsbedeutung für eine Krankheit zu erklären.

Haltung und Futter haben den größten Beitrag zur Merkmalsbedeutung über alle fünf Krankheiten hinweg.

Umweltmerkmale haben einen mäßig hohen Beitrag zu allen Diagnosen außer periparturaler Hypokalzämie.

Die Beiträge von milch- und laktationsbezogenen Merkmalen zeigen eine große Varianz mit Beiträgen, die vom Krankheitstyp abhängen: Am oberen Ende haben milch- und laktationsbezogene Merkmale einen Beitrag von 13,7 % bzw. 25,9 % zur Vorhersage der periparturalen Hypokalzämie und 6,6 % bzw. 6,3 % zur Ketose-Diagnose. Darüber hinaus tragen milchbezogene Merkmale mit 8,1 % zur Merkmalsbedeutung bei der Metritis-Vorhersage bei. Am unteren Ende haben milch- und laktationsbezogene Merkmale so gut wie keinen Beitrag zu Lahmheit und Anöstrus.

Lahmheit und Anöstrus werden dagegen erheblich vom Management beeinflusst, ebenso wie die Ketose.

Das Alter (einschließlich Parität) spielt eine große Rolle bei der Vorhersage von Lahmheit (10,9 %) und periparturaler Hypokalzämie (19,8 %) und so gut wie keine Rolle bei der Vorhersage anderer Krankheiten.

Physikalische Indikatoren, wie BCS, spielen nur eine untergeordnete Rolle bei der Permutationsbedeutung für alle Krankheiten.

Das Projekt - und damit der erfindungsgemäße Ansatz - zeigen deutlich, dass in der Nutztierhaltung der Erkenntnisgewinn bezüglich der Einflussfaktoren für das Auftreten von Nutztierkrankheiten gegenüber dem bisherigen Stand erheblich verbessert und gesteigert werden kann, indem bei der statistischen Auswertung vorhandener Daten auch Daten in harmonisierter Form miteinbezogen werden, welche bisher nicht dafür in Betracht kamen. Damit kann auf eine umweltfreundliche und effiziente Weise erheblich Mehrwert in der Viehhaltung geschaffen werden, und die Ergebnisse können zur Verbesserung des Wohlbefindens der Nutztiere genutzt werden können.

Am Beispiel Prognose der Wahrscheinlichkeit für das zukünftige Erkranken eines Nutztieres ist das erfindungsgemäße Verfahren extrem vorteilhaft, weil im Bereich Nutztierhaltung schon lange sehr viele und sehr heterogene Daten gesammelt werden, und weil ein großer Anteil der durch die Daten beschriebenen Umstände zwar einen statistischen, nicht aber einen einfach erkennbaren eindeutigen kausalen Zusammenhang mit Erkrankungsrisiken hat. Das erfindungsgemäße Verfahren eignet sich bestens dazu aus dieser Gemengelage unter wirtschaftlich gut vertretbarem Aufwand, die für die Prognosen relevanten Informationen zu identifizieren und anzuwenden.

## Patentansprüche

1. Verfahren für die Vorhersage einer Krankheit von Nutztieren unter Anwendung einer Prognoseregel (9) für jede der interessierenden Krankheiten, wobei das Erstellen der Prognoseregel (9) die folgenden Schritte umfasst,
- Anlegen einer computerbasierten Datenbank, welche eine Vielzahl von dahingehend harmonisierten Datensätzen beinhaltet, dass jeder harmonisierte Datensatz genau einem individuellen Nutztier zugeordnet ist, und jede Eintragung in dem harmonisierten Datensatz genau einer Beobachtung an diesem Nutztier zugeordnet ist, wobei das Nutztier in einem Betrieb (10) untergebracht ist, und wobei alle harmonisierten Datensätze zueinander bezüglich Parameteranzahl und Parameterthema gleich sind,
- wobei die Parameter eines harmonisierten Datensatz sowohl tierbezogene Daten als auch umweltbezogene Daten beinhalten,
- wobei aus der in der Vielzahl von harmonisierten Datensätzen enthaltenen Information durch maschinelles Lernen statistische Abhängigkeiten des Auftretens der interessierenden Krankheiten vom Vorliegen der anderen Parameter untersucht und quantifiziert werden,
- wobei für jede interessierende Krankheit die erkannten statistischen Abhängigkeiten zu einer Prognoseregel (9) zusammengefasst werden, an Hand welcher computerbasiert zu individuellen Nutztieren nach Eingabe von Eingangsparametern eine Aussage über die Wahrscheinlichkeit für das zukünftige Auftreten der Krankheit an dem Nutztier, ausgegeben wird, **dadurch gekennzeichnet, dass**
das maschinelle Lernen in zwei zeitlich nacheinander stattfindenden und zueinander unterschiedlichen Arbeitsstadien angewendet wird, wobei in dem früher stattfindenden Arbeitsstadium an den vollständigen harmonisierten Datensätzen maschinell gelernt wird, und dem später stattfindendem Arbeitsstadium an Datensätzen maschinell gelernt wird, welche gegenüber den vollständigen harmonisierten Datensätzen durch Weglassung von Parametern verkleinert sind, wobei die weggelassenen Parameter solche sind, bei denen im früheren Arbeitsstadium erkannt wurde, dass ihr Beitrag zur Prognosegüte unter einer festgelegten Mindestgrenze liegt.

2. Verfahren nach Anspruch 1, **dadurch gekennzeichnet, dass** die Parameter eines harmonisierten Datensatzes Daten bezüglich mindestens zweien der Bereiche
- Abstammung,
- körperliche Merkmale,
- aktuelle Diagnoseergebnisse
- Fütterung
- Krankheiten
- Unterbringung
- Umwelt des Betriebes
- Management (Betreuung) des Nutztieres
beinhalten.

3. Verfahren nach Anspruch 1 oder 2, **dadurch gekennzeichnet, dass** zur Befüllung der harmonisierten Datensätze mit Parameterwerten, Daten aus mindestens zwei zueinander unterschiedlichen Quellen verwendet werden.

4. Verfahren nach einem der Ansprüche 1 bis 3, **dadurch gekennzeichnet, dass**
- das maschinelle Lernen in dem früher stattfindenden Arbeitsstadium in zwei Arbeitsschritten (3, 5) wiederholt erfolgt, wobei im einen Arbeitsschritt (3) an unveränderten harmonisierten Datensätzen maschinell gelernt wird, und im anderen Arbeitsschritt (5) an Datensätzen maschinell gelernt wird, welche dahingehend gegenüber den unveränderten harmonisierten Datensätzen verändert sind, dass für einen oder mehrere Parameter die Werte welche in den einzelnen ursprünglichen Datensätzen standen, zufällig zwischen den Datensätzen verschoben sind,
- wobei gemessen wird, wie sehr sich die durch maschinelles Lernen erreichte Prognosegüte zwischen den beiden Arbeitsschritten geändert hat,
- wobei das Ausmaß der Änderung der Prognosegüte das Auswahlkriterium für die Weglassung von Parametern für den gemäß Anspruch 1 im später stattfindendem Arbeitsstadium stattfindenden maschinellen Lernvorgang darstellt.

5. Verfahren nach einem der Ansprüche 1 bis Anspruch 4, **dadurch gekennzeichnet, dass** die Prognosegüte gemessen wird, indem beim maschinellen Lernen nur an einer Teilmenge der Datensätze gelernt wird, und die Treffergenauigkeit der gefundenen Prognoseregel an einer anderen Teilmenge von Datensätzen verifiziert wird.

6. Verfahren nach einem der Ansprüche 1 bis 5, **dadurch gekennzeichnet, dass** die Nutztiere Milchkühe sind.
